# EUROPEAN PATENT APPLICATION

(11) **EP 2 402 368 A1**
(43) Date of publication of application: **04.01.2012**
(21) Application number: 10168270.6
(22) Date of filing: 02.07.2010
(51) Int. Cl.: C07K 14/47, G01N 33/68, C07K 16/18

(54) **Histone citrullinated peptides and uses thereof**

(71) Applicant: Toscana Biomarkers S.r.l., 53100 Siena (IT)
(72) Inventor: Pratesi, Federico, 53100 Siena (IT); Chelli, Mario, 53100 Siena (IT); Lolli, Francesco, 53100 Siena (IT); Paolini, Ilaria, 53100 Siena (IT); Papini, Anna Maria, 53100 Siena (IT); Rovero, Paolo, 53100 Siena (IT); Migliorini, Paola, 53100 Siena (IT); Alcaro, Maria Claudia, 53100 Siena (IT)
(74) Representative: Capasso, Olga

(57) **Abstract**

The present invention refers to citrullinated synthetic peptides derived from the histone H4 protein and their use in the diagnosis of autoimmune diseases, particularly Rheumatoid Arthritis (RA).

## Description

### Field of invention

The present invention refers to citrullinated synthetic peptides derived from the histone H4 protein and their use in the diagnosis of autoimmune diseases, particularly Rheumatoid Arthritis (RA).

### State of the art

Rheumatoid arthritis (RA) is an inflammatory joint disease characterized by an autoimmune mechanism mediated by both T- and B-cells. Different autoantibodies can be detected in the biological fluids of RA patients. Some of these autoantibodies can be used for RA diagnosis and follow up.

The best known RA biomarker is the rheumatoid factor (RF), a class of IgG or IgM antibodies directed against the Fc-region of the IgG isotype of immunoglobulins. However, RF is not a specific RA marker as it can be found also in other pathological conditions and in elderly healthy individuals. The discovery of the involvement of citrullinated epitopes in RA using anti-perinuclear factor and anti-keratin antibodies boosted the identification of novel highly specific RA biomarkers. Different citrullinated peptides and proteins have been used to set up specific and sensitive diagnostic assays allowing to recognize RA autoantibodies, thus generally named as anti-citrullinated peptide (CP) antibodies (ACPA) [US20020143143, US20070087380, EP1946109, US20070148704, W02009007846, W02009000077, W02007017556, EP1456662, WO1999028344, W02008132264]. Among all the proteins associated with RA, the following citrullinated sequences have been used in diagnostic assays: fibrin [C. Masson-Bessière et al. J. Immunol. 2001, 166, 4177], collagen II [H. Burkhardt et al. Eur. J. Immunol. 2005, 35, 1643], vimentin [E.R. Vossenaar et al. Arthritis Rheum. 2004, 50, 3485], filaggrin [US20090028885, US7445903, US6890720], and viral proteins [C. Anzilotti et al. J. Rheumatol. 2006, 33, 647; WO2004087747].

The sensitivity of the tests was increased by the use of filaggrin-derived synthetic cyclic citrullinated peptides (CCPs) [G.A. Schellekens et al. J. Clin. Invest. 1998, 101, 273] [WO1998022503] characterized by a structure that optimally exposes the citrullines for antibody binding. Presently, three generations of CCP tests have been developed: CCP1 (sensitivity 68%, specificity 98%) [G.A. Schellekens et al. Arthritis Rheum. 2000, 43, 155], CCP2 (sensitivity range 39-94%, specificity range 81-100%) [J. Avouac et al. Ann. Rheum. Dis. 2006, 65, 845], and CCP3 (sensitivity range 51-83%, specificity range 93-98%) [L. Lutteri et al. Clin. Chim. Acta 2007, 386, 76]. These RA diagnostic ELISAs are commercially available (Euro-Diagnostica, Arnhem, The Netherlands; Axis-Shield, Dundee, Scotland; INOVA, San Diego, USA).

Recently, the use of citrullinated sequences derived from proteins EBNA-1 (VCP1) and EBNA-2 (VCP2) of Epstein-Barr virus allowed the set up of diagnostic assays characterized by specificities and sensitivities comparable to the ones reported by CCP2 and CCP3 tests [F. Pratesi et al. Arthritis Rheum. 2006, 54, 733; C. Anzilotti et al. J. Rheumatol. 2006, 33, 647; WO2004087747; EP091767764]. Interestingly, the comparison of the performances of the tests based on CCP2, CCP3, VCP1, and VCP2 suggested that different citrullinated sequences can detect different subgroups of anti-CP antibodies in the same set of patients. These results confirmed the important role of the amino acid sequence surrounding citrulline residues in the formation of anti-CP epitopes, in contrast with the hypothesized exclusive role of the XG or XnonG unit (X = citrulline) [EP1693673].

Another class of proteins associated with other autoimmune diseases but not yet directly related to RA diagnosis is represented by histone proteins. Histones are constituents of the nucleosomes and are classified into six classes: H1, H2A, H2B, H3, H4, and H5. Sequences derived from H1 and H2B were used to detect autoantibodies in systemic lupus erythematosus, RA and systemic sclerosis [WO03044054]. Even if it is known that these proteins are natively deiminated and generally modified [K. Arita et al. PNAS 2006, 103, 5291; A.J.W. Zendman et al. Anal. Biochem. 2007, 369, 232], no histone citrullinated sequences have been, up to now, associated with RA. However, microarrays based on the use of modified histones (i.e. phosphorylated, methylated, acetylated, and ubiquitinated), but not citrullinated, were described for the detection of antibodies [WO07132177].

### Summary of the invention

The present invention relates to citrullinated synthetic peptides derived from the core histone protein H4 and their use for the diagnosis of autoimmune diseases, in particular, RA. The investigation of the role of histone deimination in RA led to the definition of a series of citrullinated sequences derived from H4. Such sequences are able to recognize autoantibodies specific for RA. The citrullinated peptides according to the invention derive from the amino acid sequence of the human H4, in particular from the sequence comprised between amino acid (aa) 1 to aa 50 (Swiss-Prot P62805 aa 2 to aa 51) of said sequence.

It is therefore an object of the invention an antigenically effective peptide comprising, from the amino to the carboxylic terminal, the amino acid sequence:
S G X₁ G K G G K G L G K G G A K X₂ H X₃ K V L X₄ D N I Q G I T K P A I X₅ X₆ L A X₇ X₈ G G V K X₉ I S G L I (SEQ ID No. 1, Formula I), wherein the amino acids X₁-X₉ are selected independently from an arginine residue or a citrulline residue and at least two of X₁-X₉ are a citrulline residue, or a functional fragment thereof.

The peptides as described in the invention and possessing at least two citrulline residues were demonstrated to be very suitable for the specific diagnosis of RA.

Shorter peptide sequences comprising at least seven a.a. residues and derived from H4(1-50) (Formula I) are of particular interest.

Preferably, the peptide comprises the amino acid sequence S G X₁ G K G G KGLGKGGAKX₂HX₃(aa1 1 to aa 20 of SEQ ID No. 1) or G A K X₂ H X₃ K V L X₄ D N I Q G I T K P A I (aa 14 to aa 34 of SEQ ID No. 1) or K P A I X₅ X₆ L A X₇ X₈ G G V K X₉ I S G L I (aa 31 to aa 50 of SEQ ID No. 1) wherein the amino acids X₁-X₉ are selected independently from an arginine residue or a citrulline residue, and at least two of X₁-X₇ are a citrulline residue, or a functional fragment thereof.

Preferably, the peptide comprises the sequence S G Cit G K G G K G L G K G G A K Cit H Cit K V L Cit D N I Q G I T K P A I Cit Cit L A Cit Cit G G V K Cit I S G L I (SEQ ID No. 2, Formula II) wherein Cit is a citrulline residue or a functional fragment thereof.

Preferably, the peptide comprises the sequence selected from the group of S G Cit G K G G K G L G K G G A K Cit H Cit K (aa 1 to aa 20 of SEQ ID No. 2, Formula III), G A K Cit H Cit K V L Cit D N I Q G I T K P A I (aa 14 to aa 34 of SEQ ID No. 2, Formula IV), K P A I Cit Cit L A Cit Cit G G V K Cit I S G L I (aa 31 to aa 50 of SEQ ID No. 2, Formula V) wherein Cit is a citrulline residue or a functional fragment thereof.

Preferably, the peptide as indicated above is of linear form or in the form of multimeric branched peptide or of other conjugated complex.

The multimeric branched peptide preferably consists of:
- a MAP nucleus structure;
- a linear peptide having an amino acid sequence of formula III, IV, and/or V linked through a chemical bond to each of amino terminal residues of the MAP nucleus structure, wherein the linear peptides are equal or different each others.

According to the invention a MAP nucleus structure is: 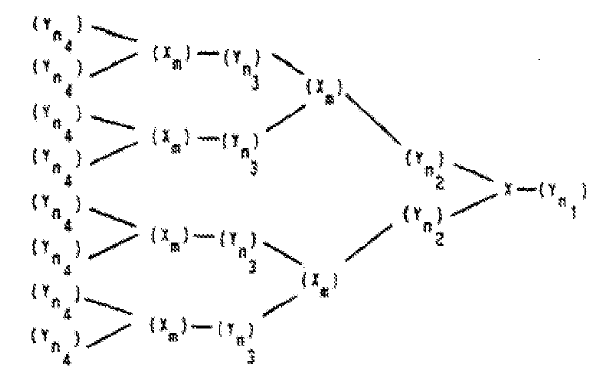 wherein X is an amino acid having at least two amino functional residues, Y is an amino acid selected from the group of alanine and/or glycine and/or a molecule used as spacer, m is 0 or 1, n₁ n₂ n₃ n₄ are integer numbers comprised between 0 and 10, and wherein bonds are carbamido bonds. A MAP or conjugate comprising a plurality of copies of a citrullinated linear synthetic peptide or of an antigenically effective fragment derived therefrom as described above, bound to an immunologically inert amino-acid core, therefore also falls within the scope of the present invention.

Still preferably, the peptide comprises four identical copies of the peptide as described above.

In a preferred embodiment the peptide is selected from the group of:
(S G Cit G K G G K G L G K G G A K Cit H Cit K)₄ K₂ K beta (Formula VI): 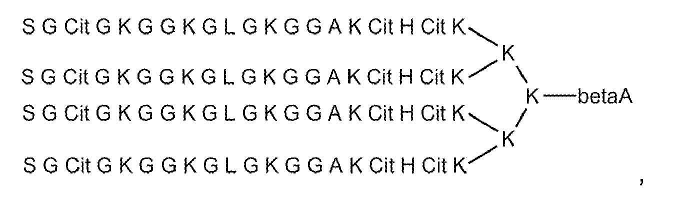
(G A K Cit H Cit K V L Cit D N I Q G I T K P A I)₄ K₂ K beta (Formula VII), 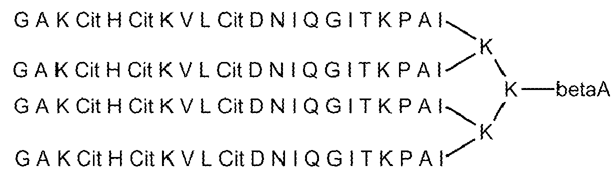 or
(K P A I Cit Cit L A Cit Cit G G V K Cit I S G L 1)₄ K₂ K beta (Formula VIII), 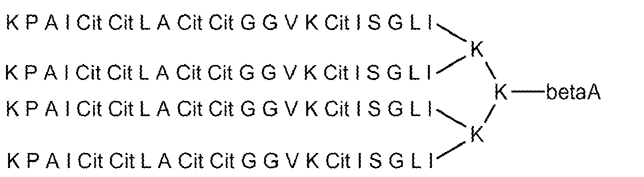
wherein Cit is a citrulline residue and betaA is a beta-alanine residue.

It is a further object of the invention a method for the diagnosis of an autoimmune disease in a subject comprising the step of detecting antibodies specific for the autoimmune disease in a biological sample by
- reacting under proper conditions said biological sample with at least one peptide of the invention to produce a complex;
- detecting the complex.

Higher sensitivity and specificity of the method is obtained when the peptides are used in the form of MAPs or of other conjugated complex, in particular in the form of tetravalent MAPs.

It was found that a method based on the use of a MAP of citrullinated peptides of formula VI, VII, or VIII gives the highest sensitivity and specificity in a test for detecting specific RA antibodies. Thus, preferably the biological sample is reacted with at least one peptide of the invention of formula III, IV, V, VI, VII, or VIII. In another preferred embodiment a method is based on the combined use of peptides of formula III, IV, and V or on the combined use of MAP of formula VII and MAP of formula VIII for detecting specific RA antibodies.

Preferably the biological sample is reacted with the peptide comprising the sequence (G A K Cit H Cit K V L Cit D N I Q G I T K P A I)₄ K₂ K betaA, 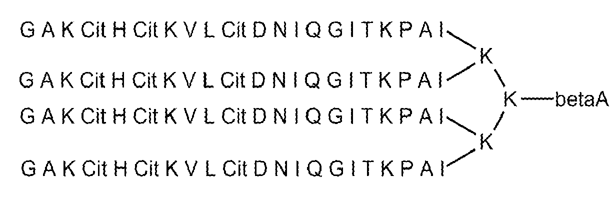 and the peptide comprising the sequence

(K P A I Cit Cit L A Cit Cit G G V K Cit I S G L)₄ K₂ K betaA, 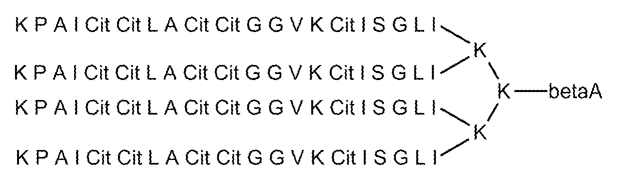 wherein Cit is a citrulline residue and betaA is a beta-alanine residue.

Still preferably the method is an immunological assay.

It is another object of the invention a kit for the diagnostic of an autoimmune disease comprising at least one peptide as indicated above, or a functional fragment thereof.

Preferably the kit comprises the peptide comprising the sequence (G A K Cit H Cit K V L Cit D N I Q G I T K P A I)₄ K₂ K betaA, 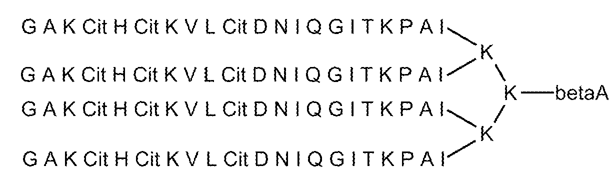 and the peptide comprising the sequence

(K P A I Cit Cit L A Cit Cit G G V K Cit I S G L I)₄ K₂ K betaA, 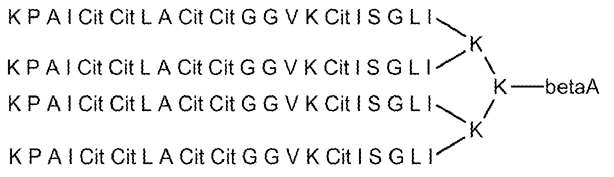 wherein Cit is a citrulline residue and betaA is a beta-alanine residue.

In particular the kit allows the detection of anti-CP antibodies in biological fluids. The kit comprises one of the citrullinated peptides as defined above or a functional fragment thereof, and at least one further reagent. Preferably, the further reagent is an anti-human immunoglobulin conjugated to an enzyme capable of reacting with a chromogenic substrate.

Preferably the method of the invention and/or the kit of the invention are for the diagnosis of rheumatoid arthritis.

It is another object of the invention an antibody directed against the peptides of the invention.

For diagnostic applications, the peptides as described in the invention can be used to detect specific antibodies present in biological fluids of RA patients. In another preferred embodiment, the peptides can be used to detect circulating and tissue-infiltrating auto-reactive B- and T-cells.

In another embodiment of the present invention, the peptides as above disclosed, bound to appropriate resins, can be used for antibody removal.

An antibody present in the biological fluids of autoimmune patients will bind to the peptides of the invention. As indicated above, the citrullinated peptides of the invention, both in linear form and in MAP form, are particularly suitable to be used as antigens in an assay to detect the presence and/or to measure the levels of autoimmune disease-specific antibodies, such as anti-CP antibodies, in a sample of a biological fluid.

In order to perform the assay, the peptides can be adsorbed or covalently linked or modified with a carrier to bind it to a solid support (e.g. chips, microspheres, gold, polystyrene, reactor vessels or wells, micro-titre plate). In a first step of the method, the sample of the biological fluid to be analyzed is placed in contact and incubated with the peptide of the invention that may be linked to the solid support. Autoimmune disease-specific antibody, such as anti-CP antibodies, that are possibly present in the sample are thus specifically bound to the peptide of the invention, producing an antigen/antibody complex. The anti-CP antibodies to be detected in the immunoassay are IgG, IgA, or IgM immunoglobulins. The evaluation of the presence and the quantity of the antigen/antibody complex can be performed with a spectroscopic, a piezoelectric, or an electrochemical biosensor.

The above described method may be an immunological assay in which an indicator antibody, like an anti-human immunoglobulin, is conjugated to an enzyme and is added to measure the antibody titer by a spectroscopic transducer.

It was found that a method based on the use of a MAP of citrullinated peptides of formula VI, VII, or VIII gives the highest sensitivity and specificity in a test for detecting specific RA antibodies. Thus, preferably the biological sample is reacted with at least one peptide of the invention of formula III, IV, V, VI, VII, or VIII. In another preferred embodiment a method is based on the combined use of peptides of formula III, IV, and V or on the combined use of MAP of formula VII and MAP of formula VIII for detecting specific RA antibodies.

In the present invention antigenically effective means that the peptide, or a functional fragment thereof is able to specifically bind autoimmune disease-specific antibodies.

### Detailed description of the invention

The invention will be now illustrated by means of the following non limiting examples.

### Example 1. Peptide Synthesis

Peptides were synthesized using a Wang resin preloaded with the C-terminal amino acid of the sequence or with the MAP core and following the Fmoc/tBu solid-phase peptide strategy [R.B. Merrifield J. Am. Chem. Soc. 1963, 85, 2149; E. Atherton *et al.* Oxford: IRL Press 1989; J.P. Tam Proc. Natl. Acad. Sci. USA 1988, 85, 5409]. Fmoc deprotections were carried out in 20 min with 20% piperidine in DMF. Coupling reactions were performed by treating the resin for 45 min with a 0.5 M solution of the Fmoc-protected amino acids and HOBt in DMF (2.5 equiv), a 0.5 M solution of TBTU in DMF (2.5 equiv), and 4 M NMM in DMF (5 equiv). Peptide cleavage from the resin and deprotection of the amino acid side chains were carried out in 3 h with TFA/thioanisole/ethanedithiol/phenol/H₂O (82.5:5:2.5:5:5). The crude products were precipitated with cold Et₂O, centrifuged, and lyophilized. The pure peptides were obtained by HPLC in a purity >95% and characterized by mass spectrometry (ESI-Orbitrap and/or MALDI-TOF).

### Example 2. ELISA for the determination of anti-citrullinated peptide antibodies

The MAP of the citrullinated peptide antigens according to the invention was diluted to a concentration of 10 µg/ml in phosphate buffered saline (PBS) and loaded into the wells of a polystyrene micro-titration plate (50 µl/well). The plate was left overnight at +4°C to permit interaction between peptide and plastics; however, it may be incubated at 37°C for 1-2 hours with the same result. Upon completion of the coating period, the wells containing the antigen, plus an equal number of wells which were used as controls, were treated for 1 hour at room temperature (RT) with 3% bovine serum albumin (BSA) in PBS. The patients' serum samples (diluted 1:200 in a buffer constituted by 1% BSA, 0.05% Tween X-100 in PBS) were then loaded onto the plate (50 µl/well) and left to incubate for 3 hours at RT. After the incubation period, one washing was performed with 1% PBS Tween X-100 and two washings were performed with PBS (150 µl/well). An anti human-IgG, IgM or IgA antibody conjugated to the enzyme alkaline phosphatase in 1% PBS BSA, 0.05% Tween X-100, was used to show that the antigen/antibody reaction had taken place. The antibody (50 µl/well) was then incubated for 3 hours at RT with agitation. Upon completion of the incubation, after three washings as described above, the alkaline phosphatase substrate (p-nitrophenyl phosphate) was added to the wells and, in the presence of the enzyme, it produced a yellow product measurable by spectrophotometric techniques at a wavelength of 405 nm; its quantity was proportional to the titre of antibodies bound. The results of the test were expressed as the percentage of positivity, calculated by dividing the absorbance of each serum sample by the absorbance of a positive serum sample the value of which was set arbitrarily at 100.

Serum samples of 97 patients suffering from RA and of 34 normal healthy subjects (NHS) were tested by this method using the peptide of formula III, IV, or V.

A result that was greater than 97.5 ^{th} percentile of the normal control sera group is defined as positive, data are reported in Table 1.

Table 1. Number of positive sera over total sera tested for peptides of formula III, IV, and V.

| Formula | RA | NHS |
|---|---|---|
| | (% RA positive sera) | |
| III | 20/97 (21%) | 2/34 |
| IV | 10/97 (10%) | 2/34 |
| V | 12/97 (12%) | 2/34 |

Serum samples of 101 patients suffering from RA and of 48 NHS were tested by this method using the MAPs of formula VI, VII, or VIII.

Moreover, 91 serum samples of disease controls other than RA (systemic sclerosis, mixed cryoglobulinemia, systemic lupus erythematosus, ankylosing spondylitis, and psoriatic arthritis) were tested by this method using MAPs of formula VI, VII or VIII, which showed the most interesting reactivities.

A result that was greater than 97.5 ^{th} percentile of the normal control sera group is defined as positive, data are reported in Table 2.

Table 2. Number of positive sera over total sera tested for MAP peptides of formula VI, VII, and VIII.

| Formula | RA positive sera | NHS | Ssc | MC | SLE | AS | PA |
|---|---|---|---|---|---|---|---|
| VI | 28/101 | 2/48 | 3/7 | 1/12 | 6/48 | 5/12 | 1/12 |
| VII | 65/101 | 2/48 | 1/7 | 0/12 | 3/48 | 0/12 | 0/12 |
| VIII | 61/101 | 1/48 | 0/7 | 0/12 | 1/48 | 1/12 | 0/12 |

| | | | | | | | |
|---|---|---|---|---|---|---|---|
| *wherein: RA = rheumatoid arthritis, NHS = normal healthy subjects, Ssc = systemic sclerosis, MC = mixed cryoglobulinemia, SLE = systemic lupus erythematosus, AS = ankylosing spondylitis, PA = psoriatic arthritis. | | | | | | | |

The overall sensitivity and specificity of the three assays are shown in the following Table 3.

Table 3. Sensitivity and specificity of the assays based on the peptide of formula VI, VII, VIII.

| Formula | Sensitivity | Specificity |
|---|---|---|
| VI | 28% | 85% |
| VII | 65% | 96% |
| VIII | 60% | 98% |

An epitope mapping of the full protein H4 was performed and the data showed that the N-terminal part, H4(1-50), comprising peptide of formula I differently citrullinated corresponded to the antigenic portion of the molecule. In particular, the citrullinated C-terminal portion of H4(1-50) is highly reactive and data reported in Tables 1-3 demonstrate that the sequences of formula III-VIII can be used to recognize RA sera.

It is noteworthy that until now, histones and their citrullination have never been associated with RA, whereas a reactivity towards antibodies present in SLE patients' sera is well known [WO03044054]. Data reported in Table 3 demonstrate a good sensitivity and specificity of the selected peptide sequences for RA. Very interestingly and surprisingly neither the MAP of formula VII nor the MAP of formula VIII showed a significant reactivity in detecting specific antibodies in SLE patients sera. Therefore, the authors of the present invention demonstrate the unexpected correlation between citullinated peptide sequences comprising the H4(1-50) region and the specific recognition of autoantibodies in RA patients sera. Therefore, such peptides are biomarkers of the disease.

In addition, the sequences object of the present invention don't overlap any one of the proteins or peptides already used to detect anti-CP antibodies (e.g. fibrin, collagen II, vimentin, filaggrin, viral proteins, and synthetic CCPs). This demonstrates the innovative role of the peptide sequences derived from H4(1-50) to recognize anti-CP antibodies with 63-67% of sensitivity and 96-97% of specificity in the case of MAPs of formula VII and VIII.

### Example 3. Diagnostic properties of an ELISA based on the contemporary use of the MAP of formula VII and of the MAP of formula VIII

To achieve a highly sensitive immunoassay, an equimolar mixture of MAP of formula VII and MAP of formula VIII was allowed to adsorb to 96-wells microtitre plates for 4 h at r.t. The test was then performed as described in example 2.

Analysing a second independent population of 147 RA patients tested for antibodies towards MAP peptides of formula VII and VIII, the authors found that:
- 87/147 (59%) RA patients recognize the peptide of formula VII;
- 89/147 (61%) RA patients recognize the peptide of formula VIII.

Analysing more in details this RA patients population positive either to peptide of formula VII or to peptide of formula VIII the authors found that:
- 71/147 (48%) react with both peptides of formula VII and VIII;
- 16/147 (11 %) sera are positive only to the peptide of formula VII;
- 18/147 (12%) sera are positive only to the peptide of formula VIII

Thus, the combined use of citrullinated peptide MAP of formula VII and citrullinated MAP of formula VIII improves the diagnostic performances of the test in respect of the single peptide based assays. The combined use leads to a RA high sensitive diagnostic assay (105/147 - 71,5% RA positive sera) as the two antibody populations identified in RA sera are overlapped but different.

### Example 4. Purification of anti-citrullinated peptides antibodies and uses thereof

Anti-CP antibodies can be purified from serum of RA patients by means of affinity chromatography procedures.

A citrullinated peptide or MAP according to the present invention was conjugated to CNBr-activated sepharose according to standard procedures known by one skilled in the art. Total immunoglobulins from sera containing anti-CP antibodies were precipitated with 50% saturated ammonium sulfate; the precipitates were dissolved in phosphate buffer (pH 7.4) and dialyzed overnight against PBS. Enriched immunoglobulin preparations were applied to the column, and the flow through was collected for subsequent analysis. The column was extensively washed with 20 mM Na₂HPO₄, 150 mM NaCl (pH 7.2), and the antibodies bound to the column were eluted by 0.1 M glycine buffer (pH 2.8) (0.5 ml/fraction), immediately neutralized with 50 µl Tris 1M (pH 8.0), and dialyzed overnight against PBS. The anti-CP antibody content in the eluates and flowthrough was tested by ELISA.

Such purified antibodies can be used as controls in solid-phase assays using citrullinated antigens.

## Claims

1. An antigenically effective peptide comprising, from the amino to the carboxylic terminal, the amino acid sequence:
SG X₁ G K G G K G L G K G G A K X₂ H X₃ K V L X₄ D N I Q G I T K P A I X₅ X₆ L A X₇ X₈ G G V K X₉ I S G L I (SEQ ID No. 1), wherein the amino acids X₁-X₉ are selected independently from an arginine residue or a citrulline residue and at least two of X₁-X₉ are a citrulline residue, or a functional fragment thereof.

2. The peptide of claim 1 comprising the amino acid sequence S G X₁ G K G G K G L G K G G A K X₂ H X₃ (aa 1 to aa 20 of SEQ ID No.1) or G A K X₂ H X₃ K V L X₄ D I Q G I K P A I (aa 14 to aa 34 of SEQ **ID** No.1) or KPAI X₅ X₆ L A X₇ X₈ G G V K X₉ I S G L I (aa 31 to aa 50 of SEQ ID No. 1) wherein the amino acids X₁-X₉ are selected independently from an arginine residue or a citrulline residue, and at least two of X₁-X₇ are a citrulline residue, or a functional fragment thereof.

3. The peptide of claim 1 comprising the sequence S G Cit G K G G K G L G K G G A K Cit H Cit K V L Cit D N I Q G I T K P A I Cit Cit L A Cit Cit G G V K Cit I S G L I (SEQ ID No. 2) wherein Cit is a citrulline residue or a functional fragment thereof.

4. The peptide of claim 2 comprising the sequence selected from the group of S G Cit G K G G K G L G K G G A K Cit H Cit K (aa 1 to aa 20 of SEQ ID No. 2), G A K Cit H Cit K V L Cit D N I Q G I T K P A I (aa 14 to aa 34 of SEQ ID No. 2), K P A I Cit Cit L A Cit Cit G G V K Cit I S G L I (aa 31 to aa 50 of SEQ ID No. 2) wherein Cit is a citrulline residue or a functional fragment thereof.

5. The peptide according to any one of preceding claims being of linear form.

6. The peptide according to any one of claim 1 to 4, being in the form of multimeric branched peptide.

7. The peptide of claim 6 comprising four identical copies of the peptide according to claim 1 to 4.

8. The peptide of claim 7 being selected from the group of: (S G Cit G K G G K G L G K G G A K Cit H Cit K)₄ K₂ K betaA, 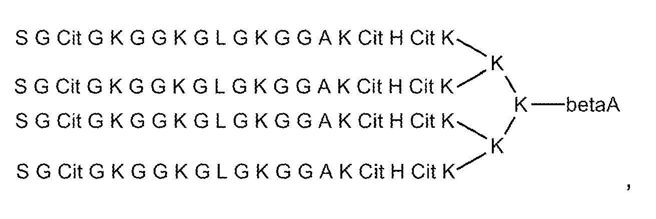 (G A K Cit H Cit K V L Cit D N I Q G I T K P A I)₄ K₂ K betaA, 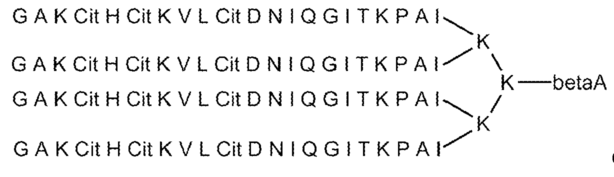 or
(K P A I Cit Cit L A Cit Cit G G V K Cit I S G L I)₄ K₂ K betaA, 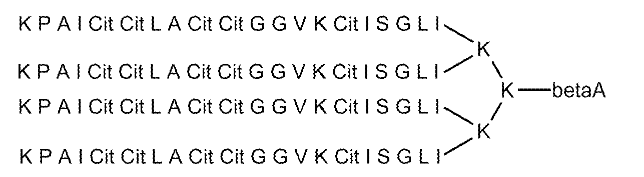 wherein Cit is a citrulline residue and betaA is a beta-alanine residue.

9. A method for the diagnosis of an autoimmune disease in a subject comprising the step of detecting antibodies specific for the autoimmune disease in a biological sample by
- reacting under proper conditions said biological sample with at least one peptide of any one of claim 1 to 8 to produce a complex;
- detecting the complex.

10. The method of claim 9 wherein the biological sample is reacted with the peptide comprising the sequence (G A K Cit H Cit K V L Cit D N I Q G I T K P A 1)₄ K₂ K betaA, 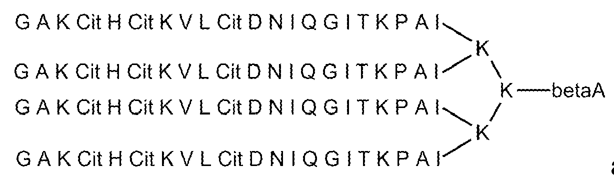 and the peptide comprising the sequence
(K P A I Cit Cit L A Cit Cit G G V K Cit I S G L I)₄ K₂ K betaA, 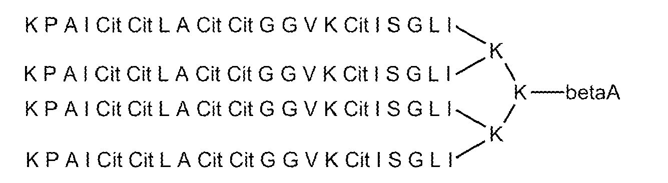 wherein Cit is a citrulline residue and betaA is a beta-alanine residue.

11. The method of any one of claims 9 or 10 being an immunological assay.

12. A kit for the diagnostic of an autoimmune disease comprising at least one peptide of any one of claim 1 to 8, or a functional fragment thereof.

13. The kit of claim 12 comprising the peptide comprising the sequence (G A K Cit H Cit K V L Cit D N I Q G I T K P A I)₄ K₂ K betaA, 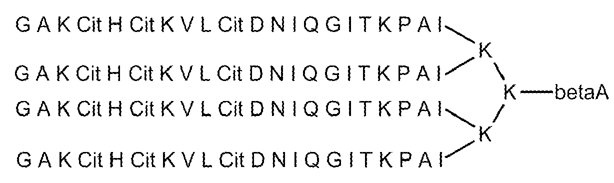 and the peptide comprising the sequence
(K P A I Cit Cit L A Cit Cit G G V K Cit I S G L I)₄ K₂ K betaA, 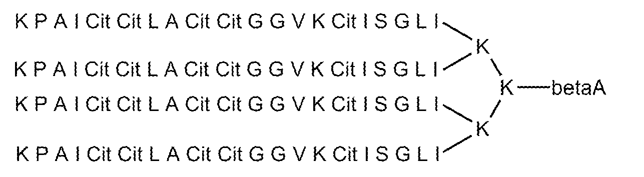 wherein Cit is a citrulline residue and betaA is a beta-alanine residue.

14. The method of claim 9 or the kit of claim 12 wherein the autoimmune disease is rheumatoid arthritis.

15. An antibody directed against the peptide of any one of claims 1 to 8.


SEQUENCE LISTING

<110>  Toscana Biomarkers srl
       PRATESI, Federico
       ALCARO, Maria Claudia
       CHELLI, Mario
       LOLLI, Francesco
       PAOLINI, Ilaria
       PAPINI, Anna Maria
       ROVERO, Paolo
       MIGLIORINI, Paola

<120>  HISTONE CITRULLINATED PEPTIDES AND USES THEREOF

<130>  BE 109193

<160>  2

<170>  PatentIn version 3.5

<210>  1
<211>  50
<212>  PRT
<213>  Artificial Sequence

<220>
<223>  synthetic peptide


<220>
<221>  MISC_FEATURE
<222>  (3)..(3)
<223>  arginine or citrulline residue

<220>
<221>  MISC_FEATURE
<222>  (17)..(17)
<223>  arginine or citrulline residue

<220>
<221>  MISC_FEATURE
<222>  (19)..(19)
<223>  arginine or citrulline residue

<220>
<221>  MISC_FEATURE
<222>  (23)..(23)
<223>  arginine or citrulline residue

<220>
<221>  MISC_FEATURE
<222>  (35)..(35)
<223>  arginine or citrulline residue

<220>
<221>  MISC_FEATURE
<222>  (36)..(36)
<223>  arginine or citrulline residue

<220>
<221>  MISC_FEATURE
<222>  (39)..(39)
<223>  arginine or citrulline residue

<220>
<221>  MISC_FEATURE
<222>  (40)..(40)
<223>  arginine or citrulline residue

<220>
<221>  MISC_FEATURE
<222>  (45)..(45)
<223>  arginine or citrulline residue

<400>  1

Ser Gly Xaa Gly Lys Gly Gly Lys Gly Leu Gly Lys Gly Gly Ala Lys
1               5                   10                  15

Xaa His Xaa Lys Val Leu Xaa Asp Asn Ile Gln Gly Ile Thr Lys Pro
            20                  25                  30

Ala Ile Xaa Xaa Leu Ala Xaa Xaa Gly Gly Val Lys Xaa Ile Ser Gly
        35                  40                  45

Leu Ile
    50

<210>  2
<211>  50
<212>  PRT
<213>  Artificial Sequence

<220>
<223>  synthetic peptide

<220>
<221>  MISC_FEATURE
<222>  (3)..(3)
<223>  citrulline residue

<220>
<221>  MISC_FEATURE
<222>  (17)..(17)
<223>  citrulline residue

<220>
<221>  MISC_FEATURE
<222>  (19)..(19)
<223>  citrulline residue

<220>
<221>  MISC_FEATURE
<222>  (23)..(23)
<223>  citrulline residue

<220>
<221>  MISC_FEATURE
<222>  (35)..(35)
<223>  citrulline residue

<220>
<221>  MISC_FEATURE
<222>  (36)..(36)
<223>  citrulline residue

<220>
<221>  MISC_FEATURE
<222>  (39)..(39)
<223>  citrulline residue

<220>
<221>  MISC_FEATURE

<222>  (40)..(40)
<223>  citrulline residue

<220>
<221>  MISC_FEATURE
<222>  (45)..(45)
<223>  citrulline residue

<400>  2

Ser Gly Xaa Gly Lys Gly Gly Lys Gly Leu Gly Lys Gly Gly Ala Lys
1               5                   10                  15

Xaa His Xaa Lys Val Leu Xaa Asp Asn Ile Gln Gly Ile Thr Lys Pro
            20                  25                  30

Ala Ile Xaa Xaa Leu Ala Xaa Xaa Gly Gly Val Lys Xaa Ile Ser Gly
        35                  40                  45

Leu Ile
    50